# EUROPEAN PATENT APPLICATION

(11) **EP 1 579 858 A1**
(43) Date of publication of application: **28.09.2005**
(21) Application number: 04007360.3
(22) Date of filing: 26.03.2004
(51) Int. Cl.: A61K 31/165, A61P 25/02

(54) **Novel use of peptide compounds for treating pain in painful diabetic neuropathy**

(71) Applicant: SCHWARZ PHARMA AG, D-40789 Monheim/Rhld. (DE)
(72) Inventor: Rauschkolb-Löffler, Christine Dr. med., 42657 Solingen (DE); Koch, Brigitte Dr. oec. troph, 40789 Monheim (DE)
(74) Representative: Weiss, Wolfgang

(57) **Abstract**

The present invention concerns the use of peptide compounds for treating pain in painful diabetic neuropathy, preferably in diabetic distal sensory polyneuropathy.

## Description

The present invention is directed to the use of a class of peptide compounds for treating pain in painful diabetic neuropathy, preferably in diabetic distal sensory polyneuropathy.

Certain peptides are known to exhibit central nervous system (CNS) activity and are useful in the treatment of epilepsy and other CNS disorders. These peptides which are described in the U.S. Patent No. 5,378,729 have the Formula (Ia): 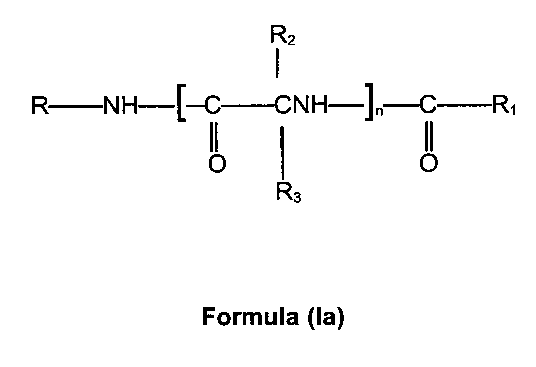 wherein
R is hydrogen, lower alkyl, lower alkenyl, lower alkynyl, aryl, aryl lower alkyl, heterocyclic, heterocyclic lower alkyl, lower alkyl heterocyclic, lower cycloalkyl, lower cycloalkyl lower alkyl, and R is unsubstituted or is substituted with at least one electron withdrawing group or electron donating group;
R₁ is hydrogen or lower alkyl, lower alkenyl, lower alkynyl, aryl lower alkyl, aryl, heterocyclic lower alkyl, heterocyclic, lower cycloalkyl, lower cycloalkyl lower alkyl, each unsubstituted or substituted with an electron donating group or an electron withdrawing group; and
R₂ and R₃ are independently hydrogen, lower alkyl, lower alkenyl, lower alkynyl, aryl lower alkyl, aryl, heterocyclic, heterocyclic lower alkyl, lower alkyl heterocyclic, lower cycloalkyl, lower cycloalkyl lower alkyl, or Z-Y wherein R₂ and R₃ may be unsubstituted or substituted with at least one electron withdrawing group or electron donating group;
Z is O, S, S(O)ₐ, NR₄, PR₄ or a chemical bond;
Y is hydrogen, lower alkyl, aryl, aryl lower alkyl, lower alkenyl, lower alkynyl, halo, heterocyclic, heterocyclic lower alkyl, and Y may be unsubstituted or substituted with an electron donating group or an electron withdrawing group, provided that when Y is halo, Z is a chemical bond, or
ZY taken together is NR₄NR₅R₇, NR₄OR₅, ONR₄R₇, OPR₄R₅, PR₄OR₅, SNR₄R₇, NR₄SR₇, SPR₄R₅ or PR₄SR₇, NR₄PR₅R₆ or PR₄NR₅R₇, 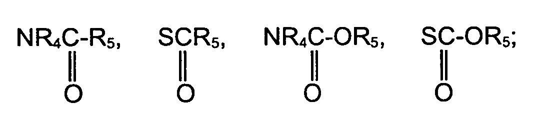 R₄, R₅ and R₆ are independently hydrogen, lower alkyl, aryl, aryl lower alkyl, lower alkenyl, or lower alkynyl, wherein R₄, R₅ and R₆ may be unsubstituted or substituted with an electron withdrawing group or an electron donating group; and
R₇ is R₆ or COOR₈ or COR₈;
R₈ is hydrogen or lower alkyl, or aryl lower alkyl, and the aryl or alkyl group may be unsubstituted or substituted with an electron withdrawing group or an electron donating group; and
n is 1-4; and
a is 1-3.

U.S. Patent No. 5,773,475 also discloses additional compounds useful for treating CNS disorders. These compounds are N-benzyl-2-amino-3-methoxy-propionamide having the Formula (IIa): 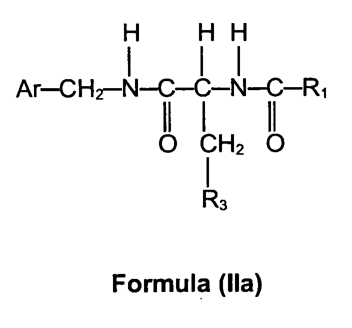 wherein
Ar is aryl which is unsubstituted or substituted with halo; R₃ is lower alkoxy; and R₁ is methyl.

The patents US 5.378.729 and US 5.773.475 are hereby incorporated by reference. However, neither of these patents describes the use of these compounds as specific analgesics for the treatment of pain in painful diabetic neuropathy, especially in diabetic distal sensory polyneuropathy.

WO 02/074297 relates to the use of a compound according to Formula (IIa) wherein Ar is phenyl which may be substituted by at least one halo, R₃ is lower alkoxy containing 1-3 carbon atoms and R₁ is methyl for the preparation of pharmaceutical compositions useful for the treatment of allodynia related to peripheral neuropathic pain.

WO 02/074784 relates to the use of a compound having Formula (Ia) or/and Formula (IIa) showing antinociceptive properties for treating different types and symptoms of acute and chronic pain, especially non neuropathic inflammatory pain, e.g. rheumatoid arthritic pain or/and secondary inflammatory osteo-arthritic pain.

Pain is a subjective experience and the perception of pain is performed in particular parts of the Central Nervous System (CNS). Usually noxious (peripheral) stimuli are transmitted to the Central Nervous System (CNS) beforehand, but pain is not always associated with nociception. A broad variety of different types of clinical pain exists, that are derived from different underlying pathophysiological mechanisms and that will need different treatment approaches.

The perception of pain may be characterized by three major types of clinical pain:
- acute pain
- chronic pain
- neuropathic pain

Acute clinical pain may result from inflammation or soft tissue injury, for instance. This type of pain is adaptive and has the biologically relevant function of warning and enabling healing and repair of an already damaged body part to occur undisturbed. A protective function is achieved by making the injured/inflamed area and surrounding tissue hypersensitive to all stimuli so that contact with any external stimulus is avoided. The neuronal mechanisms underlying this type of clinical pain are fairly well understood and pharmacological control of acute clinical pain is available and effective by means of e.g. Non-Steroidal Anti-Inflammatory Drugs (NSAIDs) up to opioids depending on type and extension of the sensation.

Chronic clinical pain appears as sustained sensory abnormalities resulting from an ongoing peripheral pathology such as cancer or chronic inflammation (e.g. arthritis) or it can be independent of the initiating triggers.

The latter being maladaptive, offering no survival advantage and very often no effective treatment is available.

There are several causes of human neuropathy with considerable variability in symptoms and neurological deficits. Painful neuropathies are defined as neurological disorders characterised by persistence of pain and hypersensitivity in a body region, of which the sensory innervation has been damaged, but damage to sensory nerves does not always produce neuropathic pain, usually loss of sensation rather than hypersensitivity or pain are observed.

Neuropathic pain can be classified as peripheral and central neuropathic pain. Peripheral neuropathic pain is caused by injury or infection of peripheral sensory nerves, whereas central neuropathic pain is caused by damage to the CNS or/and the spinal cord. Both peripheral and central neuropathic pain can occur without obvious initial nerve damage.

Common analgesics like opioids and non-steroidal anti-inflammatory drugs (NSAIDS) improve only insufficiently chronic abnormal pain syndromes as peripheral and central neuropathic pain due to insufficient efficacy or limiting side effects. In the search for alternative treatment regimes to produce satisfactory and sustained pain relief, corticosteroids, conduction blockade, glycerol, antidepressants, local anesthetics, gangliosides and electrostimulation have been tried, but mainly anti-convulsants have been found useful against various types of peripheral neuropathic pain conditions. A subset of patients with neuropathic pain responds to opioids. Gabapentin or pregabalin is effective in reducing pain in patients with diabetic neuropathy.

The mechanisms of neuropathic pain in diabetic patients are poorly understood. Current treatments use a variety of pharmacological, surgical, physical and psychological approaches. However, the evidence for many of the treatments is still limited.

Pain derived from a diabetic sensory neuropathy is the most common form of diabetic neuropathy. It is usually of insidious onset. Predominant pain may be combined with temperature and tactile loss. The pain is usually aching, prickling, or burning in quality with superimposed stabs, and often most troublesome at night. The pain is felt predominantly in the lower limbs, however, with occurence also at the upper limbs and trunk.

If general overactivity and unleaded low threshold activation of sensory neurons is considered as one of the main syndroms of neuropathy and neuropathic pain sensation with a marked mechanoallodynia as the most disabling clinical sympton, selective inhibition of this pathophysiological event instead of general inhibition of high threshold noxious stimuli (by e.g. local anesthetics) of the normal sensory nociception provides clear advantages.

The use of compounds of Formula (Ib) or/and Formula (IIb) for treatment of pain in painful diabetic neuropathy has not been reported. Thus, the present invention concerns the use of said compounds of Formulae (Ib) or/and (IIb) for the preparation of a pharmaceutical composition for the prevention, alleviation or/and treatment of diabetic pain such as pain associated with all types of painful diabetic neuropathy, especially, but not limited to, for the prevention, alleviation or/and treatment of pain associated with diabetic distal sensory polyneuropathy.

Surprisingly, application of compounds (Ib) or/and (IIb), particularly (R)-2-acetamide-N-benzyl-3-methoxypropionamide (SPM 927) exhibited a statistically significant and clinically meaningful efficacy in reducing pain in subjects with painful diabetic neuropathy in a randomized double blind placebo controlled trial. Testing of diabetic neuropathy has not been conducted previously, either in animals or in humans. This is the first time that the efficacy of SPM927 in diabetic neuropathic pain in humans has been evaluated. The patients included in the trial had clinically diagnosed pain attributed to diabetic distal sensory polyneuropathy and a diagnosis of Diabetes mellitus (Type I or Type II). The patients had good or fair diabetic control which was optimized for at least 3 months prior to the start of the trial.

In particular, when compared to placebo treated diabetic patients, diabetic patients treated with the compounds (Ib) or/and (IIb), particularly SPM927, had a reduced average daily pain, overall pain, present pain intensity and pain interference with sleep. Treatment with SPM927 was associated with an improvement in the subjects' perception of pain interference with general activity, the patients' global impression of change in pain, clinical global impression of change in pain, the subject's perception of different neuropathic pain qualities, quality of life and proportion of pain-free days. The analysis of safety data revealed no serious safety issues. In particular, no interference was observed with the treatment for diabetes control.

The invention is applicable in animals, particularly mammals including humans.

A compound according to the invention useful for the prevention, alleviation or/and treatment of pain associated with painful diabetic neuropathy, especially associated with diabetic distal sensory polyneuropathy, or/and for the prevention, alleviation or/and treatment of a condition of pain associated with painful diabetic neuropathy which is, for example, average daily pain, overall pain, present pain intensity, pain interference with sleep, the subjects' perception of pain interference with general activity, the patients' global impression of change in pain, clinical global impression of change in pain, the subject's perception of different neuropathic pain qualities, quality of life and proportion of pain-free days, has the general Formula (Ib) 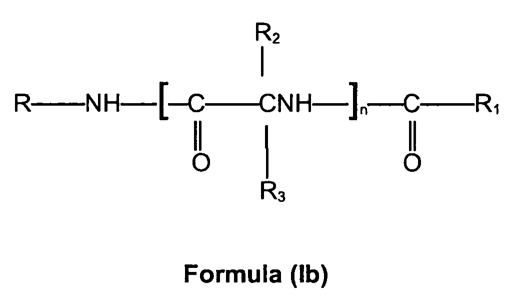 wherein
R is hydrogen, lower alkyl, lower alkenyl, lower alkynyl, aryl, aryl lower alkyl, heterocyclic, heterocyclic lower alkyl, lower alkyl heterocyclic, lower cycloalkyl or lower cycloalkyl lower alkyl, and R is unsubstituted or is substituted with at least one electron withdrawing group, and/or at least one electron donating group;
R₁ is hydrogen or lower alkyl, lower alkenyl, lower alkynyl, aryl lower alkyl, aryl, heterocyclic lower alkyl, lower alkyl heterocyclic, heterocyclic, lower cycloalkyl, lower cycloalkyl lower alkyl, each unsubstituted or substituted with at least one electron donating group and/or at least one electron withdrawing group;
and
R₂ and R₃ are independently hydrogen, lower alkyl, lower alkenyl, lower alkynyl, aryl lower alkyl, aryl, halo, heterocyclic, heterocyclic lower alkyl, lower alkyl heterocyclic, lower cycloalkyl, lower cycloalkyl lower alkyl, or Z-Y wherein R₂ and R₃ may be unsubstituted or substituted with at least one electron withdrawing group and/or at least one electron donating group;
Z is O, S, S(O)ₐ, NR₄, NR'₆, PR₄ or a chemical bond;
Y is hydrogen, lower alkyl, aryl, aryl lower alkyl, lower alkenyl, lower alkynyl, halo, heterocyclic, heterocyclic lower alkyl, lower alkyl heterocyclic and Y may be unsubstituted or substituted with at least one electron donating group and/or at least one electron withdrawing group, provided that when Y is halo, Z is a chemical bond, or
ZY taken together is NR₄NR₅R₇, NR₄OR₅, ONR₄R₇, OPR₄R₅, PR₄OR₅, SNR₄R₇, NR₄SR₇, SPR₄R₅, PR₄SR₇, NR₄PR₅R₆, PR₄NR₅R₇ or N⁺R₅R₆R₇, 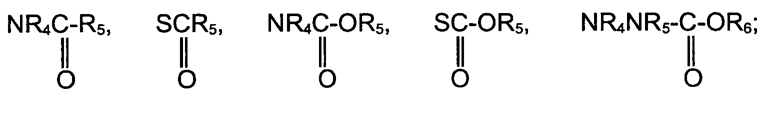 R'₆ is hydrogen, lower alkyl, lower alkenyl, or lower alkenyl which may be unsubstituted or substituted with at least one electron withdrawing group or/and at least one electron donating group;
R₄, R₅ and R₆ are independently hydrogen, lower alkyl, aryl, aryl lower alkyl, lower alkenyl, or lower alkynyl, wherein R₄, R₅ and R₆ may independently be unsubstituted or substituted with at least one electron withdrawing group or/and at least one electron donating group;
R₇ is R₆ or COOR₈ or COR₈, which R₇ may be unsubstituted or substituted with at least one electron withdrawing group or/and at least one electron donating group;
R₈ is hydrogen or lower alkyl, or aryl lower alkyl, and the aryl or alkyl group may be unsubstituted or substituted with at least one electron withdrawing group or/and at least one electron donating group; and
n is 1-4; and
a is 1-3.

Preferably the compound according has the general Formula (IIb) 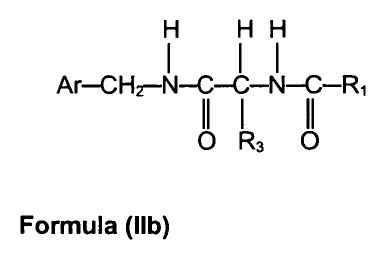 wherein
Ar is aryl, especially phenyl, which is unsubstituted or substituted with at least one halo; R₃ is -CH₂-Q, wherein Q is lower alkoxy; and R₁ is lower alkyl, especially methyl.

The present invention is also directed to a pharmaceutical composition comprising a compound according to Formula (Ib) or/and Formula (IIb) useful for the prevention, alleviation or/and treatment of pain associated with painful diabetic neuropathy, especially associated with diabetic distal sensory polyneuropathy, or/and for the prevention, alleviation or/and treatment of a condition of pain associated with painful diabetic neuropathy which is, for example, average daily pain, overall pain, present pain intensity, pain interference with sleep, the subjects' perception of pain interference with general activity, the patients' global impression of change in pain, clinical global impression of change in pain, the subject's perception of different neuropathic pain qualities, quality of life and proportion of pain-free days, and to the preparation of said pharmaceutical composition.

The compounds of Formula (la) are described in U.S. Patent No. 5,378,729, the contents of which are incorporated by reference.

The "lower alkyl" groups when used alone or in combination with other groups, are lower alkyl containing from 1 to 6 carbon atoms, especially 1 to 3 carbon atoms, and may be straight chain or branched. These groups include methyl, ethyl, propyl, isopropyl, butyl, isobutyl, tertiary butyl, amyl, hexyl, and the like.

The "lower alkoxy" groups are lower alkoxy containing from 1 to 6 carbon atoms, especially 1 to 3 carbon atoms, and may be straight chain or branched. These groups include methoxy, ethoxy, propoxy, butoxy, isobutoxy, tert-butoxy, pentoxy, hexoxy and the like.

The "aryl lower alkyl" groups include, for example, benzyl, phenylethyl, phenylpropyl, phenylisopropyl, phenylbutyl, diphenylmethyl, 1,1-diphenylethyl, 1,2-diphenylethyl, and the like.

The term "aryl", when used alone or in combination, refers to an aromatic group which contains from 6 up to 18 ring carbon atoms and up to a total of 25 carbon atoms and includes the polynuclear aromatics. These aryl groups may be monocyclic, bicyclic, tricyclic or polycyclic and are fused rings. A polynuclear aromatic compound as used herein, is meant to encompass bicyclic and tricyclic fused aromatic ring systems containing from 10-18 ring carbon atoms and up to a total of 25 carbon atoms. The aryl group includes phenyl, and the polynuclear aromatics e.g., naphthyl, anthracenyl, phenanthrenyl, azulenyl and the like. The aryl group also includes groups like ferrocenyl. Aryl groups may be unsubstituted or mono or polysubstituted with electron withdrawing or/and electron donating groups as described below.

"Lower alkenyl" is an alkenyl group containing from 2 to 6 carbon atoms and at least one double bond. These groups may be straight chained or branched and may be in the Z or E form. Such groups include vinyl, propenyl, 1-butenyl, isobutenyl, 2-butenyl, 1-pentenyl, (Z)-2-pentenyl, (E)-2-pentenyl, (Z)-4-methyl-2-pentenyl, (E)-4-methyl-2-pentenyl, pentadienyl, e.g., 1, 3 or 2,4-pentadienyl, and the like.

The term "lower alkynyl" is an alkynyl group containing 2 to 6 carbon atoms and may be straight chained as well as branched. It includes such groups as ethynyl, propynyl, 1-butynyl, 2-butynyl, 1-pentynyl, 2-pentynyl, 3-methyl-1-pentynyl, 3-pentynyl, 1-hexynyl, 2-hexynyl, 3-hexynyl and the like.

The term "lower cycloalkyl" when used alone or in combination is a cycloalkyl group containing from 3 to 18 ring carbon atoms and up to a total of 25 carbon atoms. The cycloalkyl groups may be monocyclic, bicyclic, tricyclic, or polycyclic and the rings are fused. The cycloalkyl may be completely saturated or partially saturated. Examples include cyclopropyl, cyclobutyl, cyclopentyl, cyclohexyl, cycloheptyl, cyclooctyl, cyclodecyl, cyclohexenyl, cyclopentenyl, cyclooctenyl, cycloheptenyl, decalinyl, hydroindanyl, indanyl, fenchyl, pinenyl, adamantyl, and the like. Cycloalkyl includes the cis or trans forms. Cycloalkyl groups may be unsubstituted or mono or polysubstituted with electron withdrawing or/and electron donating groups as described below. Furthermore, the substituents may either be in endo or exo positions in the bridged bicyclic systems.

The term "electron-withdrawing and electron donating" refer to the ability of a substituent to withdraw or donate electrons, respectively, relative to that of hydrogen if the hydrogen atom occupied the same position in the molecule. These terms are well understood by one skilled in the art and are discussed in Advanced Organic Chemistry, by J. March, John Wiley and Sons, New York, NY, pp.16-18 (1985) and the discussion therein is incorporated herein by reference. Electron withdrawing groups include halo, including bromo, fluoro, chloro, iodo and the like; nitro, carboxy, lower alkenyl, lower alkynyl, formyl, carboxyamido, aryl, quaternary ammonium, haloalkyl such as trifluoromethyl, aryl lower alkanoyl, carbalkoxy and the like. Electron donating groups include such groups as hydroxy, lower alkoxy, including methoxy, ethoxy and the like; lower alkyl, such as methyl, ethyl, and the like; amino, lower alkylamino, di(loweralkyl) amino, aryloxy such as phenoxy, mercapto, lower alkylthio, lower alkylmercapto, disulfide (lower alkyldithio) and the like. One of ordinary skill in the art will appreciate that some of the aforesaid substituents may be considered to be electron donating or electron withdrawing under different chemical conditions. Moreover, the present invention contemplates any combination of substituents selected from the above-identified groups.

The term "halo" includes fluoro, chloro, bromo, iodo and the like.

The term "acyl" includes lower alkanoyl containing from 1 to 6 carbon atoms and may be straight chains or branched. These groups include, for example, formyl, acetyl, propionyl, butyryl, isobutyryl, tertiary butyryl, pentanoyl and hexanoyl.

As employed herein, a heterocyclic group contains at least one sulfur, nitrogen or oxygen ring atom, but also may include several of said atoms in the ring. The heterocyclic groups contemplated by the present invention include heteroaromatics and saturated and partially saturated heterocyclic compounds. These heterocyclics may be monocyclic, bicyclic, tricyclic or polycyclic and are fused rings. They may preferably contain up to 18 ring atoms and up to a total of 17 ring carbon atoms and a total of up to 25 carbon atoms. The heterocyclics are also intended to include the so-called benzoheterocyclics. Representative heterocyclics include furyl, thienyl, pyrazolyl, pyrrolyl, methylpyrrolyl, imidazolyl, indolyl, thiazolyl, oxazolyl, isothiazolyl, isoxazolyl, piperidyl, pyrrolinyl, piperazinyl, quinolyl, triazolyl, tetrazolyl, isoquinolyl, benzofuryl, benzothienyl, morpholinyl, benzoxazolyl, tetrahydrofuryl, pyranyl, indazolyl, purinyl, indolinyl, pyrazolindinyl, imidazolinyl, imadazolindinyl, pyrrolidinyl, furazanyl, N-methylindolyl, methylfuryl, pyridazinyl, pyrimidinyl, pyrazinyl, pyridyl, epoxy, aziridino, oxetanyl, azetidinyl, the N-oxides of the nitrogen containing heterocycles, such as the N-oxides of pyridyl, pyrazinyl, and pyrimidinyl and the like. Heterocyclic groups may be unsubstituted or mono or polysubstituted with electron withdrawing or/and electron donating groups.

The preferred heterocyclics are thienyl, furyl, pyrrolyl, benzofuryl, benzothienyl, indolyl, methylpyrrolyl, morpholinyl, pyridiyl, pyrazinyl, imidazolyl, pyrimidinyl, or pyridazinyl. The preferred heterocyclic is a 5 or 6-membered heterocyclic compound. The especially preferred heterocyclic is furyl, pyridyl, pyrazinyl, imidazolyl, pyrimidinyl, or pyridazinyl. The most preferred heterocyclics are furyl and pyridyl.

The preferred compounds are those wherein n is 1, but di (n=2), tri (n=3) and tetrapeptides (n=4) are also contemplated to be within the scope of the invention.

The preferred values of R is aryl lower alkyl, especially benzyl especially those wherein the phenyl ring thereof is unsubstituted or substituted with electron donating groups or/and electron withdrawing groups, such as halo (e.g., F).

The preferred R₁ is H or lower alkyl. The most preferred R₁ group is methyl.

The preferred electron donating substituents or/and electron withdrawing substituents are halo, nitro, alkanoyl, formyl, arylalkanoyl, aryloyl, carboxyl, carbalkoxy, carboxamido, cyano, sulfonyl, sulfoxide, heterocyclic, guanidine, quaternary ammonium, lower alkenyl, lower alkynyl, sulfonium salts, hydroxy, lower alkoxy, lower alkyl, amino, lower alkylamino, di(loweralkyl) amino, amino lower alkyl, mercapto, mercaptoalkyl, alkylthio, and alkyldithio. The term "sulfide" encompasses mercapto, mercapto alkyl and alkylthio, while the term disulfide encompasses alkyldithio. Especially preferred electron donating or/and electron withdrawing groups are halo or lower alkoxy, most preferred are fluoro or methoxy. These preferred substituents may be present on any one of the groups in Formula (Ib) or/and (IIb), e.g. R, R₁, R₂, R₃, R₄, R₅ R₆, R'₆, R₇ and/or R₈ as defined herein.

The ZY groups representative of R₂ and R₃ include hydroxy, alkoxy, such as methoxy, ethoxy, aryloxy, such as phenoxy; thioalkoxy, such as thiomethoxy, thioethoxy; thioaryloxy such as thiophenoxy; amino; alkylamino, such as methylamino, ethylamino; arylamino, such as anilino; lower dialkylamino, such as, dimethylamino; trialkyl ammonium salt, hydrazino; alkylhydrazino and arylhydrazino, such as N-methylhydrazino, N-phenylhydrazino, carbalkoxy hydrazino, aralkoxycarbonyl hydrazino, aryloxycarbonyl hydrazino, hydroxylamino, such as N-hydroxylamino (-NH-OH), lower alkoxy amino [(NHOR₁₈) wherein R₁₈ is lower alkyl], N-lower alkylhydroxyl amino [(NR₁₈)OH wherein R₁₈ is lower alkyl], N-lower alkyl-O-lower alkylhydroxyamino, i.e., [N(R₁₈)OR₁₉ wherein R₁₈ and R₁₉ are independently lower alkyl], and o-hydroxylamino (-O-NH₂); alkylamido such as acetamido; trifluoroacetamido; lower alkoxyamino, (e.g., NH(OCH₃); and heterocyclicamino, such as pyrazoylamino.

The preferred heterocyclic groups representative of R₂ and R₃ are monocyclic 5- or 6-membered heterocyclic moieties of the formula: 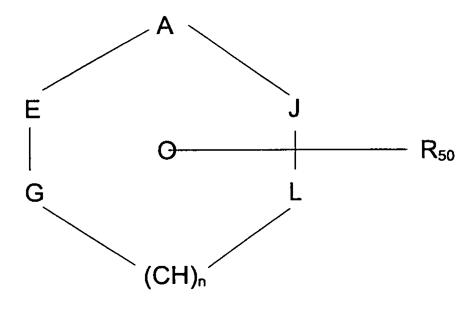 or those corresponding partially or fully saturated form thereof wherein n is 0 or 1; and
R₅₀ is H or an electron withdrawing group or electron donating group;
A, E, L, J and G are independently CH, or a heteroatom selected from the group consisting of N, O, S;
but when n is 0, G is CH, or a heteroatom selected from the group consisting of NH, O and S with the proviso that at most two of A, E, L, J and G are heteroatoms.

When n is 0, the above heteroaromatic moiety is a five membered ring, while if n is 1, the heterocyclic moiety is a six membered monocyclic heterocyclic moiety. The preferred heterocyclic moieties are those aforementioned heterocyclics which are monocyclic.

If the ring depicted hereinabove contains a nitrogen ring atom, then the N-oxide forms are also contemplated to be within the scope of the invention.

When R₂ or R₃ is a heterocyclic of the above formula, it may be bonded to the main chain by a ring carbon atom. When n is 0, R₂ or R₃ may additionally be bonded to the main chain by a nitrogen ring atom.

Other preferred moieties of R₂ and R₃ are hydrogen, aryl, e.g., phenyl, aryl alkyl, e.g., benzyl and alkyl.

It is to be understood that the preferred groups of R₂ and R₃ may be unsubstituted or mono or poly substituted with electron donating or/and electron withdrawing groups. It is preferred that R₂ and R₃ are independently hydrogen, lower alkyl, which is either unsubstituted or substituted with electron withdrawing groups or/and electron donating groups, such as lower alkoxy (e.g., methoxy, ethoxy, and the like), N-hydroxylamino, N-lower alkylhydroxyamino, N-loweralkyl-O-loweralkyl and alkylhydroxyamino.

It is preferred that one of R₂ and R₃ is hydrogen.

It is preferred that n is one.

It is more prefered that n=1 and one of R₂ and R₃ is hydrogen. It is especially preferred that in this embodiment, R₂ is hydrogen and R₃ is lower alkyl or ZY; Z is O, NR₄ or PR₄; Y is hydrogen or lower alkyl; ZY is NR₄NR₅R₇, NR₄OR₅, ONR₄R₇,

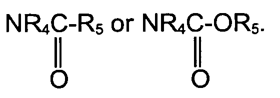

In another especially preferred embodiment, n=1, R₂ is hydrogen and R₃ is lower alkyl which may be substituted or unsubstituted with an electron donating or electron withdrawing group, NR₄OR₅, or ONR₄R₇,

In yet another especially preferred embodiment, n = 1, R₂ is hydrogen and R₃ is lower alkyl which is unsubstituted or substituted with hydroxy or loweralkoxy, NR₄OR₅ or ONR₄R₇, wherein R₄, R₅ and R₇ are independently hydrogen or lower alkyl, R is aryl lower alkyl, which aryl group may be unsubstituted or substituted with an electron withdrawing group and R₁ is lower alkyl. In this embodiment it is most preferred that aryl is phenyl, which is unsubstituted or substituted with halo.

It is preferred that R₂ is hydrogen and R₃ is hydrogen, an alkyl group which is unsubstituted or substituted by at least an electron donating or electron withdrawing group or ZY. In this preferred embodiment, it is more preferred that R₃ is hydrogen, an alkyl group such as methyl, which is unsubstituted or substituted by an electron donating group, or NR₄OR₅ or ONR₄R₇, wherein R₄, R₅ and R₇ are independently hydrogen or lower alkyl. It is preferred that the electron donating group is lower alkoxy, and especially methoxy or ethoxy.

It is preferred that R₂ and R₃ are independently hydrogen, lower alkyl, or ZY; Z is O, NR₄ or PR₄;
Y is hydrogen or lower alkyl or
ZY is NR₄R₅R₇, NR₄OR₅, ONR₄R₇,

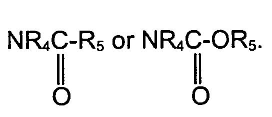

It is also preferred that R is aryl lower alkyl. The most preferred aryl for R is phenyl. The most preferred R group is benzyl. In a preferred embodiment, the aryl group may be unsubstituted or substituted with an electron donating or electron withdrawing group. If the aryl ring in R is substituted, it is most preferred that it is substituted with an electron withdrawing group, especially on the aryl ring. The most preferred electron withdrawing group for R is halo, especially fluoro.

The preferred R₁ is lower alkyl, especially methyl.

It is more preferred that R is aryl lower alkyl and R₁ is lower alkyl.

Further preferred compounds are compounds of Formula (Ib) wherein n is 1; R₂ is hydrogen; R₃ is hydrogen, a lower alkyl group, especially methyl which is substituted by an electron donating or electron withdrawing group or ZY; R is aryl, aryl lower alkyl, such as benzyl, wherein the aryl group is unsubstituted or substituted with an electron donating or electron withdrawing group and R₁ is lower alkyl. In this embodiment, it is more preferred that R₃ is hydrogen, a lower alkyl group, especially methyl, which may be substituted by electron donating group, such as lower alkoxy, (e.g., methoxy, ethoxy and the like), NR₄OR₅ or ONR₄R₇ wherein these groups are defined hereinabove.

The most preferred compounds utilized are those of the Formula (IIb): 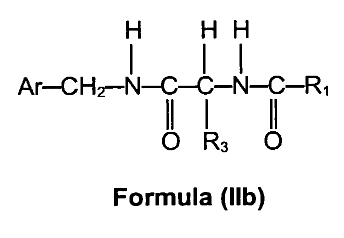 wherein
Ar is aryl, especially phenyl, which is unsubstituted or substituted with at least one electron donating group or electron withdrawing group, especially halo,
R₁ is lower alkyl, especially containing 1-3 carbon atoms; and
R₃ is as defined herein, but especially hydrogen, loweralkyl, which is unsubstituted or substituted by at least an electron donating group or electron withdrawing group or ZY. It is even more preferred that R₃ is, in this embodiment, hydrogen, an alkyl group which is unsubstituted or substituted by an electron donating group, NR₄OR₅ or ONR₄R₇. It is most preferred that R₃ is CH₂-Q, wherein Q is lower alkoxy, especially containing 1-3 carbon atoms; NR₄OR₅ or ONR₄R₇ wherein R₄ is hydrogen or alkyl containing 1-3 carbon atoms, R₅ is hydrogen or alkyl containing 1-3 carbon atoms, and R₇ is hydrogen or alkyl containing 1-3 carbon atoms.

The most preferred R₁ is CH₃. The most preferred R₃ is CH₂-Q, wherein Q is methoxy.

The most preferred aryl is phenyl. The most preferred halo is fluoro.

The most preferred compounds include:
(R)-2-acetamido-N-benzyl-3-methoxy-propionamide;
O-methyl-N-acetyl-D-serine-m-fluorobenzyl-amide;
O-methyl-N-acetyl-D-serine-p-fluorobenzyl-amide;
N-acetyl-D-phenylglycine benzylamide;
D-1,2-(N,O-dimethylhydroxylamino)-2-acetamide acetic acid benzylamide;
D-1,2-(O-methylhydroxylamino)-2-acetamido acetic acid benzylamide.

It is to be understood that the various combinations and permutations of the Markush groups of R₁, R₂, R₃, R and n described herein are contemplated to be within the scope of the present invention. Moreover, the present invention also encompasses compounds and compositions which contain one or more elements of each of the Markush groupings in R₁, R₂, R₃, n and R and the various combinations thereof. Thus, for example, the present invention contemplates that R₁ may be one or more of the substituents listed hereinabove in combination with any and all of the substituents of R₂, R₃, and R with respect to each value of n.

The compounds utilized in the present invention may contain one or more asymmetric carbons and may exist in racemic and optically active forms. The configuration around each asymmetric carbon can be either the D or L form. It is well known in the art that the configuration around a chiral carbon atoms can also be described as R or S in the Cahn-Prelog-Ingold nomenclature system. All of the various configurations around each asymmetric carbon, including the various enantiomers and diastereomers as well as racemic mixtures and mixtures of enantiomers, diastereomers or both are contemplated by the present invention.

In the principal chain, there exists asymmetry at the carbon atom to which the groups R₂ and R₃ are attached. When n is 1, the compounds of the present invention is of the formula 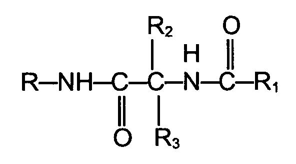 wherein R, R₁, R₂, R₃, R₄, R₅, R₆, R'₆, R₇, R₈, R₅₀ Z and Y are as defined previously.

As used herein, the term configuration shall refer to the configuration around the carbon atom to which R₂ and R₃ are attached, even though other chiral centers may be present in the molecule. Therefore, when referring to a particular configuration, such as D or L, it is to be understood to mean the D or L stereoisomer at the carbon atom to which R₂ and R₃ are attached. However, it also includes all possible enantiomers and diastereomers at other chiral centers, if any, present in the compound.

The compounds of the present invention are directed to all the optical isomers, i.e., the compounds of the present invention are either the L-stereoisomer or the D-stereoisomer (at the carbon atom to which R₂ and R₃ are attached). These stereoisomers may be found in mixtures of the L and D stereoisomer, e.g., racemic mixtures. The D stereoisomer is preferred.

Depending upon the substituents, the present compounds may form addition salts as well. All of these forms are contemplated to be within the scope of this invention including mixtures of the stereoisomeric forms

The manufacture of the utilized compounds is described in U.S. Patent Nos. 5,378,729 and 5,773.475, the contents of both of which are incorporated by reference.

The compounds utilized in the present invention are useful as such as depicted in the Formulae (Ib) or/and (IIb) or can be employed in the form of salts in view of its basic nature by the presence of the free amino group. Thus, the compounds of Formulae (Ib) or/and (IIb) forms salts with a wide variety of acids, inorganic and organic, including pharmaceutically acceptable acids. The salts with therapeutically acceptable acids are of course useful in the preparation of formulation where enhanced water solubility is most advantageous.

These pharmaceutically acceptable salts have also therapeutic efficacy. These salts include salts of inorganic acids such as hydrochloric, hydroiodic, hydrobromic, phosphoric, metaphosphoric, nitric acid and sulfuric acids as well as salts of organic acids, such as tartaric, acetic, citric, malic, benzoic, perchloric, glycolic, gluconic, succinic, aryl sulfonic, (e.g., p-toluene sulfonic acids, benzenesulfonic), phosphoric, malonic, and the like.

It is preferred that the compound utilized in the present invention is used in therapeutically effective amounts.

The physician will determine the dosage of the present therapeutic agents which will be most suitable and it will vary with the form of administration and the particular compound chosen, and furthermore, it will vary with the patient under treatment, the age of the patient, the type of malady being treated. He will generally wish to initiate treatment with small dosages substantially less than the optimum dose of the compound and increase the dosage by small increments until the optimum effect under the circumstances is reached. When the composition is administered orally, larger quantities of the active agent will be required to produce the same effect as a smaller quantity given parenterally. The compounds are useful in the same manner as comparable therapeutic agents and the dosage level is of the same order of magnitude as is generally employed with these other therapeutic agents.

In a preferred embodiment, the compounds of the present invention are administered in amounts ranging from about 1 mg to about 10 mg per kilogram of body weight per day. This dosage regimen may be adjusted by the physician to provide the optimum therapeutic response. Patients in need thereof may be treated with doses of the compound of the present invention of at least 50 mg/day, preferably of at least 200 mg/day, more preferably of at least 300 mg/day and most preferably of at least 400 mg/day. At the maximum, a patient in need thereof may be treated with doses at a maximum of 3 g/day, more preferably a maximum of 1 g/day and most preferably a maximum of 600 mg/day.

In another preferred embodiment, the daily doses are increased until a predetermined daily dose is reached which is maintained during the further treatment.

In yet another preferred embodiment, several divided doses may be administered daily. For example, three doses per day may be administered, preferably two doses per day. It is more preferred to administer a single dose per day.

In yet another preferred embodiment, an amount of the compounds of the present invention may be administered which results in a plasma concentration of 0.1 to 15 µg/ml (trough) and 5 to 18.5 µg/ml (peak), calculated as an average over a plurality of treated subjects.

The compounds of Formulae (Ib) or/and (IIb) may be administered in a convenient manner, such as by oral, intravenous (where water soluble), intramuscular, intrathecal or subcutaneous routes. Oral administration is preferred.

The pharmaceutical composition of the present invention may be prepared for the treatment regimen as described above, in particular for the treatment with doses as described above, to effect plasma concentrations as described above, for administration periods or/and administration routes as specified in the embodiments of the present invention as described above.

In another preferred embodiment, the method of the present invention as described above for the treatment of a mammal including a human being in need thereof comprises administering a compound of the present invention in combination with administering an active agent for the prevention, alleviation or/and treatment of Diabetes mellitus Type I or Type II. The compound of the present invention and the active agent for the preparation, alleviation or/and treatment of Diabetes mellitus may be administered together, i.e. in a single dose form, or may be administered separately, i.e. in a separate dose form. Thus, the pharmaceutical composition of the present invention may comprise a compound of the present invention as defined above and may further comprise an active agent for the prevention, alleviation or/and treatment of Diabetes mellitus Type I or Type II. The pharmaceutical composition may comprise a single dose form or may comprise a separate dose form comprising a first composition comprising a compound of the present invention as defined above and a second composition for the prevention, alleviation, or/and treatment of Diabetes mellitus Type I or Type II.

The compounds of the present invention may be used for the preparation of a pharmaceutical composition as described above.

The compounds of Formulae (Ib) or/and (IIb) may be orally administered, for example, with an inert diluent or with an assimilable edible carrier, or it may be enclosed in hard or soft shell gelatin capsules, or it may be compressed into tablets, or it may be incorporated directly into the fool of the diet. For oral therapeutic administration, the active compound of Formulae (Ib) or/and (IIb) may be incorporated with excipients and used in the form of ingestible tablets, buccal tablets, troches, capsules, elixirs, suspensions, syrups, wafers, and the like. Such compositions and preparations should contain at least 1 % of active compound of Formulae (Ib) or/and (IIb). The percentage of the compositions and preparations may, of course, be varied and may conveniently be between about 5 to about 80 % of the weight of the unit. The amount of active compound of Formulae (Ib) or/and (IIb) in such therapeutically useful compositions is such that a suitable dosage will be obtained. Preferred compositions or preparations according to the present invention contains between about 10 mg and 6 g active compound of Formulae (Ib) or/and (IIb).

The tablets, troches, pills, capsules and the like may also contain the following: A binder such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, lactose or saccharin may be added or a flavoring agent such as peppermint, oil of wintergreen, or cherry flavoring. When the dosage unit form is a capsule, it may contain, in addition to materials of the above type, a liquid carrier.

Various other materials may be present as coatings or otherwise modify the physical form of the dosage unit. For instance, tablets, pills, or capsules may be coated with shellac, sugar or both. A syrup or elixir may contain the active compound, sucrose as a sweetening agent, methyl and propylparabens as preservatives, a dye and flavoring such as cherry or orange flavor. Of course, any material used in preparing any dosage unit form should be pharmaceutically pure and substantially non-toxic in the amounts employed. In addition, the active compound may be incorporated into sustained-release preparations and formulations. For example, sustained release dosage forms are contemplated wherein the active ingredient is bound to an ion exchange resin which, optionally, can be coated with a diffusion barrier coating to modify the release properties of the resin.

The active compound may also be administered parenterally or intraperitoneally. Dispersions can also be prepared in glycerol, liquid, polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions (where water soluble) or dispersions and sterile powders for the extemporaneous preparation of sterile injectable solutions or dispersions. In all cases the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating such as lecithin, by the maintenance of the required particle size in the case of dispersions and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active compound in the required amount in the appropriate solvent with various of the other ingredients enumerated above, as required, followed by filtered sterilization. Generally, dispersions are prepared by incorporating the various sterilized active ingredient into a sterile vehicle which contains the basic dispersion medium and the required other ingredients from those enumerated above. In the case of sterile powders for the preparation of sterile injectable solutions, the preferred methods of preparation are vacuum drying the freeze-drying technique plus any additional desired ingredient from previously sterile-filtered solution thereof.

As used herein, "pharmaceutically acceptable carrier" includes any and all solvents, dispersion media, coatings, antibacterial and antifungal agent, isotonic and absorption delaying agents for pharmaceutical active substances as well known in the art. Except insofar as any conventional media or agent is incompatible with the active ingredient, its use in the therapeutic compositions is contemplated. Supplementary active ingredients can also be incorporated into the compositions.

It is especially advantageous to formulate parenteral compositions in dosage unit form or ease of administration and uniformity of dosage. Dosage unit form as used herein refers to physically discrete units suited as unitary dosages for the mammalian subjects to be treated; each unit containing a predetermined quantity of active material calculated to produce the desired therapeutic effect in association with the required pharmaceutical carrier. The specifics for the novel dosage unit forms of the invention are dictated by and directly dependent on (a) the unique characteristics of the active material an the particular therapeutic effect to be achieved, and (b) the limitations inherent in the art of compounding such as active material for the treatment of disease in living subjects having a diseased condition in which bodily health is impaired as herein disclosed in detail.

The principal active ingredient is compounded for convenient and effective administration in effective amounts with a suitable pharmaceutically acceptable carrier in dosage unit form as hereinbefore described. A unit dosage form can, for example, contain the principal active compound in amounts ranging from about 10 mg to about 6 g. Expressed in proportions, the active compound is generally present in from about 1 to about 750 mg/ml of carrier. In the case of compositions containing supplementary active ingredients, the dosages are determined by reference to the usual dose and manner of administration of the said ingredients.

As used herein the term "patient" or "subject" refers to a warm blooded animal, and preferably mammals, such as, for example, cats, dogs, horses, cows, pigs, mice, rats and primates, including humans. The preferred patient is humans.

The term "treat" refers to either relieving the pain associated with a disease or condition or alleviating the patient's disease or condition.

The compounds of the present invention are administered to a patient suffering from the aforementioned type of pain in an analgesic effective amount. These amounts are equivalent to the therapeutically effective amounts described hereinabove.

The following example shows the properties of SPM 927 in reducing pain in a clinical trial in subjects with painful diabetic neuropathy, in particular with diabetic distal sensory neuropathy.

The used substance was SPM 927 which is the synonym for Harkoseride. The standard chemical nomenclature is (R)-2-acetamide-N-benzyl-3-methoxypropionamide.

### Example

A randomized, double-blind placebo controlled trial to investigate safety and efficacy of SPM 927 in painful diabetic neuropathy was formed.

**Objectives:** The primary objective of the study was to determine whether SPM 927 was effective in reducing pain in subjects with diabetic distal sensory polyneuropathy.
Secondary objectives were the following:
- To investigate how SPM 927 affects different qualities of neuropathic pain
- To investigate whether SPM 927 affects sleep and activity in subjects suffering from diabetic distal sensory polyneuropathy
- To investigate whether SPM 927 influences the Quality of Life and the Profile of Mood States
- To further investigate the tolerability and safety of SPM 927
- To investigate the pre- and post-dose plasma concentrations of unchanged SPM 927

**Methodology:** This was a multicenter, double-blind, placebo-controlled trial to assess the efficacy, safety, tolerability, and pre- and post-dose plasma concentrations of oral SPM 927 in subjects with painful diabetic neuropathy.

Baseline data were collected during the last week of the 4-week Run-In Phase to ensure subject eligibility. Eligible subjects were then randomized to receive a maximum of 400 mg per day of SPM 927 (starting at 100 mg/day for 3 weeks, then titrating up at 100 mg intervals for 3 weeks) or placebo. The highest attained dose was maintained for 4 weeks during the Maintenance Phase, after which subjects entered the Taper Phase and were tapered off of study medication for 1 week. The Taper Phase was followed by a 2-week Safety Follow-Up Phase.

**Number of subjects (planned and analyzed):** A total of 140 subjects were planned to be enrolled in order to achieve 100 evaluable subjects.
A total of 438 subjects were screened for this trial. Two hundred seventy-seven were screen failures due to stringent entry criteria, and 42 were Run-In failures. Therefore, a total of 119 subjects were randomized. All 119 subjects who were randomized also received at least one dose of trial medication and are referred to as the Safety Set (SS). All randomized subjects also had at least one post-Baseline efficacy assessment and are considered part of the Full Analysis Set (FAS). Ninety-three subjects in the FAS completed the Maintenance Phase and did not have a major protocol violation and are, therefore, considered part of the Per Protocol Set (PPS). A total of 94 subjects completed all phases of the trial.

**Diagnosis and main criteria for inclusion:** Subjects were male or female, age 18 or older. Subjects had clinically diagnosed pain attributed to diabetic distal sensory polyneuropathy for 1-5 years and a diagnosis of diabetes mellitus (Type I or Type II). Subjects had at least moderate pain (mean pain intensity during the Baseline week ≥4 out of 10 on an 11-point Likert scale) which had been stable for 4 weeks prior to randomization. Furthermore, subjects had good or fair diabetic control (glycosylated hemoglobin [HbA_{1c}] <10%), which was optimized (best effort to achieve best control) for at least three months prior to Visit 1.

**Test product, dose and mode of administration, batch number:** Subjects took 50 mg and 100 mg SPM 927 tablets (Schwarz Pharma AG, Germany).

**Duration of treatment:** After completion of screening assessments, subjects began a 4-week Run-In Phase. Eligible subjects were randomized at Visit 3 in a 1:1 ratio to active SPM 927 or matching placebo starting with 100 mg/day (50 mg twice daily [BID]) for three weeks. Following the titration scheme, their dosage was escalated by 100 mg in weekly increments to a maximum dose of 400 mg/day (provided tolerability was satisfactory).

The dose was up-titrated only if tolerability of the previous dose level was satisfactory. In the event that subjects experienced adverse events such that, in the investigators judgment, the dose of SPM 927 should not be up-titrated, subjects were permitted to either remain at their current dose level or back-titrate to their previous dose level. Only one back-titration was permitted during the trial. Once the dose of trial medication had been reduced, it could not be re-escalated.

Once subjects had completed the Titration Phase (i.e., after 5 weeks), subjects entered the 4-week Maintenance Phase.

If subjects reached total or sufficient pain relief with lower doses than 400 mg/day, after careful consideration by the investigator, they were allowed to stay on the attained dose level in the Maintenance Phase. If adverse events were intolerable during the Titration Phase or the first week of the Maintenance Phase (only for those subjects reaching the 400 mg/day level), subjects could have been down-titrated, once, to the next lowest dose. Subjects were treated at 400 mg/day (or highest dose achieved) for 4 weeks in the Maintenance Phase.

At the end of the Maintenance Phase, subjects entered the Taper Phase and were tapered off the active medication or placebo in a blinded manner (over a period of 1 week). Subjects on 400 mg/day decreased their trial medication by 200 mg during the Taper Phase. Subjects on 300 mg/day reduced their dose of trial medication by 100 mg during the Taper Phase. Subjects on 100 mg/day and 200 mg/day received placebo during the Taper Phase.

**Reference therapy, dose and mode of administration:** Placebo was provided in matching tablets.

### Criteria for evaluation:

**Efficacy:** The primary variable was the within-subject change in average daily pain score from the Baseline week to Maintenance Phase, using an 11-point Likert scale (0-10).

Secondary variables included the following:
- Within-subject change in average daily pain score from the Baseline week to the third week of titration
- Within-subject change in average daily pain score from the Baseline week to each week of the trial
- Within-subject change in average daily Present Pain Intensity from the Baseline week to each week of the trial
- Change in subject's perception of different neuropathic pain qualities (Neuropathic Pain Scale [NPS]) assessed at Visits 1, 3, 6, 10, 11, and 12
- Change in subject's perception of pain interference with sleep and activity from the Baseline week to each week of the trial. Sleep was assessed every morning (Likert - out of Brief Pain Inventory [BPI]), and activity was assessed every evening (Likert - out of BPI)
- Change in subject's perception of pain assessed at every clinic visit, as measured by Short Form - McGill Pain Questionnaire (SF-MPQ) in 3 sections:

- Categorical pain rating scale, from 0 (no pain) to 3 (severe pain)
- Visual Analog Scale (VAS), rating pain on 100-mm scale
- Present Pain Intensity (PPI), rating pain on a 6-point scale
- Patient's global impression of change in pain (PGIC) completed at Visits 6, 10, and 12
- Clinical global impression of change in pain (CGIC) completed at Visits 6, 10, and 12
- Change in Short Form-36 (SF-36) Quality of Life (QOL) questionnaire completed at Visits 1, 3, 6, 10, and 12
- Change in Profile of Mood States (POMS) questionnaire completed at Visits 1, 3, 6, 10, and 12
- Proportion of pain-free days during Baseline, Titration Phase, Maintenance Phase, and Taper Phase
- Use (number) of rescue medication

**Pharmacokinetics:** Plasma concentrations of unchanged SPM 927 predose (trough) and peak (i.e., 2-4 hours post-dose) concentrations were evaluated.

**Safety:** Safety variables evaluated included adverse events, clinical laboratory assessments, electrocardiograms (ECGs), vital sign measurements, and physical and neurological examinations.

**Statistical methods:** It was determined that 46 evaluable subjects in each treatment group would yield an 80% probability of detecting a significant difference at a two-sided 5% level of significance if the true treatment effect was 1.25 units on the Likert scale with a common standard deviation of 2.11. A total of 70 subjects in each treatment group were to be accrued to allow for subjects who dropped out during the 4-week Run-In Phase or could not be evaluated because of missing Baseline or follow-up observations. It was estimated that 140 subjects would be required to enroll in order to achieve a total of 120 randomized subjects and 100 evaluable subjects for the primary analysis. If the dropout rate was lower than expected, the enrollment was to be stopped before a total of 70 subjects per treatment group were accrued.

For the primary efficacy variable, an analysis of covariance (ANCOVA) with terms for treatment and investigator type was used to compare the difference between active treatment and placebo using the Baseline Likert pain score as a covariate and the change from average Baseline to average Maintenance Phase Likert score as the response. The treatment difference was estimated on the basis of least squares mean (LSMean). A two-sided 95% confidence interval (CI) for the treatment difference was calculated. As a secondary analysis, treatment-by-investigator interaction and/or other potential factors (age, race, sex, and Baseline severity) were explored in the ANCOVA model.

The main effect ANCOVA model was applied to the change in average daily Likert pain score from Baseline to the first three weeks of the Titration Phase, to the entire Titration Phase, to the Treatment Phase, and to each visit using the Baseline value as a covariate.

### Results

### Efficacy results:

Efficacy results from this study consistently demonstrated a statistically significant difference between SPM 927 and placebo in subjects with painful diabetic neuropathy with regard to the primary efficacy endpoints tested in this trial. The reduction in mean pain scores following the administration of SPM 927 can be regarded as clinically meaningful. Analysis of the secondary efficacy endpoints provided additional statistically significant and clinically relevant results (e.g., clinically meaningful improvements in subjects' quality of sleep and daily routine activity levels).

The primary efficacy variable for this trial was the within-subject change in the average daily pain score from the Baseline week to the Maintenance Phase using an 11-point Likert scale (0-10), where Baseline was the 7-day period between Visits 2 and 3. In the Full Analysis Set (Last Observation Carried Forward) there was a 3.11-point reduction in pain from Baseline to the Maintenance Phase following SPM 27 treatment compared with a 2.21-point reduction in pain following placebo treatment based on the LSMean. The difference in LSMean pain score between the two groups (0.9) was statistically significant (p=0.0390) and clinically meaningful. Analysis of the FAS (As Observed) and PPS populations for the change from Baseline to the Maintenance Phase also demonstrated greater reductions in pain following treatment with SPM 927 than with placebo; these differences were statistically significant and clinically meaningful.

For the FAS (LOCF) and, to an even greater extent the FAS (As Observed), SPM 927 was more effective in reducing pain by visit than placebo. By the end of the first 3 weeks of Titration, SPM 27-treated subjects had lower average daily pain scores than placebo-treated subjects. As the dose of SPM 927 was escalated through the remainder of the Titration Phase and eventually stabilized during the Maintenance Phase, average daily pain scores were increasingly lower relative to placebo-treated subjects. Tapering SPM 927 was associated with subsequent increases in average daily pain score.

Changes in the secondary efficacy endpoints were consistent with those seen in the primary endpoint and provide further support for the efficacy of SPM 927 in painful diabetic neuropathy. Statistically significant differences from Baseline to the Maintenance Phase following SPM 927 treatment versus placebo were seen for the VAS (rating of overall pain) and present pain intensity of the SF-MPQ, subject's perception of pain interference with sleep, and subject's perception of pain interference with general activity. Statistically significant differences were also observed following SPM 927 and placebo treatment for CGIC and PGIC scores at the end of Maintenance Phase (Visit 10). A larger proportion of subjects in the SPM 927 group compared with the placebo group experienced a decrease of 2 or more points on the Likert pain scale during all phases of the trial. In addition, treatment with SPM 927 was associated with greater improvements than placebo in other pain indices (present pain intensity and subject's perception of different neuropathic pain qualities), quality of life data (SF-36 and POMS), and proportion of pain-free days.

### Pharmacokinetics results:

Trough and peak plasma drug concentrations during the Maintenance Phase showed a wide range of values, explained in part by the variability in subject weight and the low number of samples collected. At the 400 mg/day dose, the mean plasma drug concentrations increased from 7.7 µg/mL to 11.4 µg/mL (trough to peak, Visit 9) and from 7.9 µg/mL to 9.1 µg/mL (Visit 10).

### Safety results:

The mean duration of exposure to study medication was 62.7 days for subjects in the placebo treatment group and 59.6 days for subjects in the SPM 927 treatment group indicating high tolerability to SPM 927.

The percentage of subjects who reported at least one treatment-emergent adverse event (TEAE) was higher in the SPM 927 treatment group, with overall incidence rates of 87% of 60 subjects in the SPM 927 group and 75% of 59 subjects in the placebo group.

Among all subjects, TEAEs were most common in the central and peripheral nervous system, with 46 subjects (39% of 119 subjects) reporting at least one adverse event in this body system. However, adverse events associated with the central and peripheral nervous system were reported by comparable percentages of subjects in each treatment group (39% of 59 placebo subjects, 38% of 60 SPM 927 subjects). Review of the adverse event profile provided no evidence for an adverse effect on any particular body system.

Overall, headache (20% of 119 subjects), dizziness (12%), accident not otherwise specified (NOS) (11%), upper respiratory tract infection (10%), and nausea (9%) were the most frequently reported TEAEs. In general, the proportions of subjects who reported specific TEAEs, or who reported TEAEs associated with particular body systems, were comparable between the placebo and SPM 927 treatment groups.

Overall, considerably more subjects first reported AEs during the Titration Phase compared with the Maintenance Phase and Taper/Safety Follow-Up Phase. For most body systems, the number of subjects reporting AEs within each body system was greater during the Titration Phase compared with the Maintenance Phase and Taper/Safety Follow-Up Phase.

No subjects died during this study. A total of 2 subjects reported 2 serious adverse events during this study: one subject with one serious adverse event (SAE) in the placebo treatment group (pain in right hip judged to be not related to study medication) and one subject with one SAE in the SPM 927 treatment group (abnormal electrocardiogram [ECG] judged to be unlikely related to study medication). A total of 8 subjects (7% of 119 subjects) withdrew from the trial due to an AE: 3 in the placebo group (5% of 59 subjects) and 5 in the SPM 927 group (8% of 60 subjects).

Mean and median changes from baseline in hematology, clinical chemistry and coagulation, urinalysis, vital signs, and ECG parameters were small, within normal ranges, comparable between treatment groups, and not of clinical concern. No safety issues with regards to QT or PR interval were identified during the study. HbA_{1c} levels changed only slightly from Baseline to Visit 8 for the two treatment groups (mean changes of -0.1 % and 0.1% for the placebo and SP 927 groups, respectively). No important differences were observed in these parameters in the transitions from Baseline to on-therapy values between the two treatment groups. Neurological examination findings at the end of the trial compared with those at Baseline did not suggest any significant effects due to SPM 927.

### Conclusions:

In summary, SPM 927 showed statistically significant and clinically meaningful efficacy in reducing neuropathic pain due to diabetic distal sensory polyneuropathy when titrated to a maintenance dose of 400 mg/day. Overall, 60 subjects with painful diabetic neuropathy were treated with SP 927 100-400 mg/day for up to 82 days; 46 of these subjects completed all phases of the trial. Analyses of safety data (adverse events, clinical laboratory evaluations, ECGs, vital signs, and physical examinations) revealed no serious safety issues and support the further clinical development of SPM 927 as an agent to treat diabetic patients with peripheral neuropathic pain.

## Claims

1. Use of a compound having the Formula (Ib) 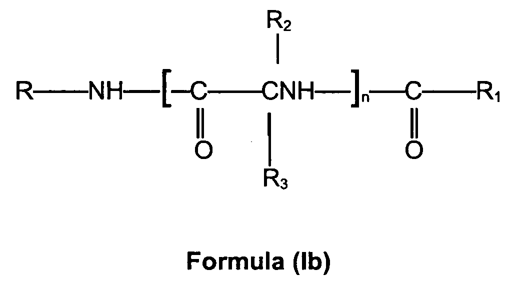 wherein
R is hydrogen, lower alkyl, lower alkenyl, lower alkynyl, aryl, aryl lower alkyl, heterocyclic, heterocyclic lower alkyl, lower alkyl heterocyclic, lower cycloalkyl or lower cycloalkyl lower alkyl, and R is unsubstituted or is substituted with at least one electron withdrawing group or/and at least one electron donating group;
R₁ is hydrogen or lower alkyl, lower alkenyl, lower alkynyl, aryl lower alkyl, aryl, heterocyclic lower alkyl, lower alkyl heterocyclic, heterocyclic, lower cycloalkyl, lower cycloalkyl lower alkyl, each unsubstituted or substituted with at least one electron donating group or/and at least one electron withdrawing group;
R₂ and R₃ are independently hydrogen, lower alkyl, lower alkenyl, lower alkynyl, aryl lower alkyl, aryl, halo, heterocyclic, heterocyclic lower alkyl, lower alkyl heterocyclic, lower cycloalkyl, lower cycloalkyl lower alkyl, or Z-Y wherein R₂ and R₃ may be unsubstituted or substituted with at least one electron withdrawing group or/and at least one electron donating group; and wherein heterocyclic in R₂ and R₃ is furyl, thienyl, pyrazolyl, pyrrolyl, methylpyrrolyl, imidazolyl, indolyl, thiazolyl, oxazolyl, isothiazolyl, isoxazolyl, piperidyl, pyrrolinyl, piperazinyl, quinolyl, triazolyl, tetrazolyl, isoquinolyl, benzofuryl, benzothienyl, morpholinyl, benzoxazolyl, tetrahydrofuryl, pyranyl, indazolyl, purinyl, indolinyl, pyrazolindinyl, imidazolinyl, imidazolindinyl, pyrrolidinyl, furazanyl, N-methylindolyl, methylfuryl, pyridazinyl, pyrimidinyl, pyrazinyl, pyridyl, epoxy, aziridino, oxetanyl, azetidinyl or, when N is present in the heterocyclic, an N-oxide thereof;
Z is O, S, S(O)ₐ, NR₄, NR₆' or PR₄ or a chemical bond;
Y is hydrogen, lower alkyl, aryl, aryl lower alkyl, lower alkenyl, lower alkynyl, halo, heterocyclic, heterocyclic lower alkyl, lower alkyl heterocyclic and Y may be unsubstituted or substituted with at least one electron donating group or/and at least one an electron withdrawing group, wherein heterocyclic has the same meaning as in R₂ or R₃ and, provided that when Y is halo, Z is a chemical bond, or ZY taken together is NR₄NR₅R₇, NR₄OR₅, ONR₄R₇, OPR₄R₅, PR₄OR₅, SNR₄R₇, NR₄SR₇, SPR₄R₅, PR₄SR₇, NR₄PR₅R₆, PR₄NR₅R₇, or N⁺R₅R₆R₇, 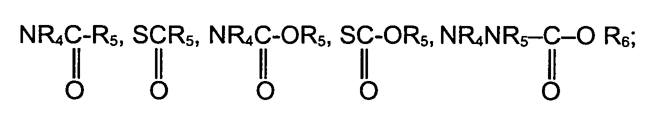 R₆' is hydrogen, lower alkyl, lower alkenyl, or lower alkynyl which may be unsubstituted or substituted with at least one electron withdrawing group or/and at least one electron donating group;
R₄, R₅ and R₆ are independently hydrogen, lower alkyl, aryl, aryl lower alkyl, lower alkenyl, or lower alkynyl, wherein R₄, R₅ and R₆ may independently be unsubstituted or substituted with at least one electron withdrawing group or/and at least one electron donating group; and
R₇ is R₆ or COOR₈ or COR₈, which R₇ may be unsubstituted or substituted with at least one electron withdrawing group or/and at least one electron donating group;
R₈ is hydrogen or lower alkyl, or aryl lower alkyl, and the aryl or alkyl group may be unsubstituted or substituted with at least one electron withdrawing group or/and at least one electron donating group; and
n is 1-4; and
a is 1-3,
or of a pharmaceutically acceptable salt thereof,
for the preparation of a pharmaceutical composition useful for the prevention, alleviation or/and treatment of pain associated with painful diabetic neuropathy.

2. Use according to claim 1, wherein the pain associated with painful diabetic neuropathy is a pain associated with diabetic distal sensory polyneuropathy.

3. Use according to any one of claims 1-2 wherein one of R₂ and R₃ is hydrogen.

4. Use according to any one of claims 1-3 wherein n is 1.

5. Use according to any one of claims 1-4 wherein one of R₂ and R₃ is hydrogen and n is 1.

6. Use according to any one of claims 1-5 wherein R is aryl lower alkyl and R₁ is lower alkyl.

7. Use according to any one of claims 1-6 wherein
R₂ and R₃ are independently hydrogen, lower alkyl, or ZY;
Z is O, NR₄ or PR₄;
Y is hydrogen or lower alkyl or
ZY is NR₄NR₅R₇, NR₄OR₅, ONR₄R₇,

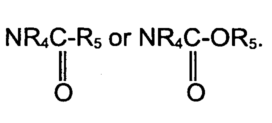

8. Use according to claim 7 wherein R₂ is hydrogen and and R₃ is lower alkyl, or ZY;
Z is O, NR₄ or PR₄;
Y is hydrogen or lower alkyl;
ZY is NR₄NR₅R₇, NR₄OR₅, ONR₄R₇,

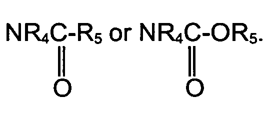

9. Use according any one of claims 1-8 wherein R₂ is hydrogen and R₃ is lower alkyl, which may be substituted or unsubstituted with at least one electron donating group or/and at least one electron withdrawing group, NR₄OR₅, or ONR₄R₇.

10. Use according to any one of claims 1-9 wherein R₃ is lower alkyl which is unsubstituted or substituted with hydroxy or loweralkoxy, NR₄OR₅ or ONR₄R₇, wherein R₄, R₅ and R₇ are independently hydrogen or lower alkyl, R is aryl lower alkyl, which aryl group may be unsubstituted or substituted with at least one electron withdrawing group and R₁ is lower alkyl.

11. Use according to any one of claims 1-10 wherein aryl is phenyl and is unsubstituted or substituted with halo.

12. Use according to any of claims 1-11 wherein the compound is
(R)-2-acetamido-N-benzyl-3-methoxy-propionamide;
O-methyl-N-acetyl-D-serine-m-fluorobenzylamide;
O-methyl-N-acetyl-D-serine-p-fluorobenzylamide;
N-acetyl-D-phenylglycinebenzylamide;
D-1,2-(N, O-dimethylhydroxylamino)-2-acetamide acetic acid benzylamide; or
D-1,2-(O-methylhydroxylamino)-2-acetamido acetic acid benzylamide.

13. Use of any one of claims 1-12 wherein the compound has the Formula (lib) 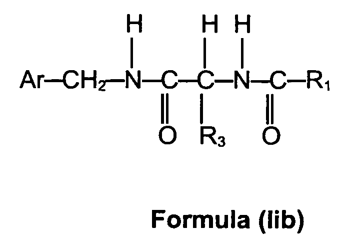 wherein
Ar is phenyl which is unsubstituted or substituted with at least one halo group;
R₃ is CH₂-Q, wherein Q is lower alkoxy containing 1-3 carbon atoms and R₁ is lower alkyl containing 1-3 carbon atoms
or of a pharmaceutically acceptable salt thereof.

14. Use according to claim 13 wherein Ar is unsubstituted phenyl.

15. Use according to claims 13 or 14 wherein halo is fluoro.

16. Use according to claims 13 to 15 wherein R₃ is CH₂-Q, wherein Q is alkoxy containing 1-3 carbon atoms and Ar is unsubstituted phenyl.

17. Use of any one of claims 1-16 wherein the compund is in the R configuration and has the formula 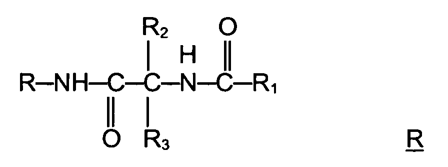 wherein
Ar is phenyl which is unsubstituted or substituted with at least one halo group;
R₃ is CH₂ -Q, wherein Q is lower alkoxy containing 1-3 carbon atoms and R₁ is methyl
or a pharmaceutically acceptable salt thereof.

18. Use according to claim 17 which is substantially enantiopure.

19. Use according to claims 17 or 18 wherein Ar is unsubstituted phenyl.

20. Use according to claims 17 to 19 wherein halo is fluoro.

21. Use according to claims 17 to 20 wherein R₃ is CH₂-Q, wherein Q is alkoxy containing 1-3 carbon atoms and Ar is unsubstituted phenyl.

22. Use according to claims 1 or 2, wherein the compound of Formula (Ib) is (R)-2-Acetamido-N-benzyl-3-methoxypropionamide or a pharmaceutically acceptable salt thereof.

23. Use according to claim 22 wherein the compund is substantially enantiopure.

24. Use according to any one of the preceding claims, wherein the pharmaceutical composition is prepared for treatment with doses of the compound of at least 100 mg/day, preferably of at least 200 mg/day, more preferably of at least 300 mg/day, most preferably of at least 400 mg/day.

25. Use according to any one of the preceding claims, wherein the pharmaceutical composition is prepared for treatment with doses of the compound at a maximum of 6 g/day, preferably at a maximum of 3 g/day, more preferably of at a maximum 1 g/day and most preferably of at a maximum of 400 mg/day.

26. Use according to any one of the preceding claims, wherein the pharmaceutical composition is prepared for treatment with increasing daily doses until a predetermined daily dose is reached which is maintained during the further treatment.

27. Use according to any one of the preceding claims, wherein the pharmaceutical composition is prepared for treatment in three doses per day, preferably two doses per day, more preferably in a single dose per day.

28. Use according to any one of the preceding claims, wherein the pharmaceutical composition is prepared for an administration resulting in a plasma concentration of 0.1 to 15 µg/ml (trough) and 5 to 18.5 µg/ml (peak), calculated as an average over a plurality of treated subjects.

29. Use according to any one of the preceding claims, wherein the pharmaceutical composition is prepared for treatment for at least one week, preferably at least two weeks, more preferably at least four weeks, most preferably at least eight weeks.

30. Use according to any one of the preceding claims, wherein the pharmaceutical composition is prepared for oral administration.

31. Use according to any one of the preceding claims wherein the pharmaceutical composition is useful for the prevention, alleviation or/and treatment of a condition associated with painful diabetic neuropathy which is average daily pain, overall pain, present pain intensity, pain interference with sleep, the subjects' perception of pain interference with general activity, the patients' global impression of change in pain, clinical global impression of change in pain, the subject's perception of different neuropathic pain qualities, quality of life and proportion of pain-free days.

32. Use according to any one of the preceding claims, wherein the pharmaceutical composition further comprises an active agent for the prevention, alleviation or/and treatment of Diabetes mellitus Type I or II.

33. Use according to claim 32 wherein the pharmaceutical composition comprises a single dose form or comprises a separate dose form comprising a first composition comprising a compound as defined in any of the claims 1 and 3 to 23 and a second composition for the prevention, alleviation or/and the treatment of Diabetes mellitus Type I or Type II.

34. Use according to any one of the preceding claims wherein the pharmaceutical composition is prepared for administration in mammals.

35. Use according to claim 34 wherein the pharmaceutical composition is prepared for administration in humans.

36. A pharmaceutical composition comprising
(a) a compound as defined in any of the claims 1 and 3 to 23, and
(b) an active agent for the prevention, alleviation or/and treatment of Diabetes mellitus Type I or Type II.

37. The pharmaceutical composition according to claim 36 which is a single dose form or comprises a separate dose form comprising a first composition comprising a compound as defined in any of the claims 1 and 3 to 23 and a second composition for the prevention, alleviation or/and the treatment of Diabetes mellitus Type I or Type II.
